# EUROPEAN PATENT APPLICATION

(11) **EP 2 208 769 A1**
(43) Date of publication of application: **21.07.2010**
(21) Application number: 08837859.1
(22) Date of filing: 10.10.2008
(51) Int. Cl.: C09K 3/00, A61K 8/28, A61K 8/29, A61K 8/89, A61K 8/898, A61Q 1/00

(54) **THICKENER/GELLANT FOR OILY SUBSTANCE**

(30) Priority: 12.10.2007 JP 2007290143
(71) Applicant: Mizutani, Genzo, Kanagawa 235-0016 (JP); Watanabe, Akihisa, Chiba 272-0835 (JP)
(72) Inventor: Mizutani, Genzo, Kanagawa 235-0016 (JP); Watanabe, Akihisa, Chiba 272-0835 (JP)
(74) Representative: Blodig, Wolfgang
(86) International application number: PCT/JP2008/068428
(87) International publication number: WO 2009/048125

(57) **Abstract**

The purpose of the invention is to provide a thickening and gelling agent that is easily dispersed into an oily substance such as a silicone oil in a small addition amount, is free of liquid separation, stably forms a thickened material or a gel state, and can prepare a thickened product or a gel having spreadability and free of sticky feeling and dry feeling. The invention provides a thickening and gelling agent for an oily substance containing the components (A) a modified product of alkoxytitanium or alkoxyzirconium with a carboxylic acid and a hydroxy compound, and (B) at least one silicone oil selected from a silicone oil having an amino group and a silicone oil having a carboxyl group; an oily composition and a cosmetic preparation, containing the thickening and gelling agent.

## Description

### Technical Field

The present invention relates to a thickening and gelling agent for an oily substance. Particularly, the invention relates to a thickening and gelling agent for an oily substance, which can sufficiently be dispersed in an oily substance containing a silicone oil and can stably thicken or gelatinize the oily substance.

### Background Art

Many thickening and gelling agents are used in industrial fields of paints, putty, adhesives, pharmaceutical preparations, inks, lubricants, cosmetics, foods and the like. Above all, a gelling agent capable of exhibiting gelation function in an aqueous system has been extensively studied for long periods of time, and has been used. For example, the conventional gelling agent includes xanthane gum, alginate and acrylic derivatives.

On the other hand, examples of a gelling agent in an oil system include 12-hydroxystearic acid, dextrin fatty acid, cholesterol derivatives, cyclic dipeptide, N-acyliamino acid amide, glycerin fatty acid ester, metallic soaps and organomodified clay. However, those gelling agents are not always satisfactory in the points of compatibility with an oily substance, gelation effect, texture of a gel obtained, viscosity of a gel obtained, separation of a liquid, change with the passage of time, and the like.

In particular, the effect on a silicone oil as an oily substance is quite insufficient.

That is, a silicone oil is an oily substance which is relatively chemically inactive, has small intermolecular force, is rich in flexibility, has low surface tension, and has properties of water repellency and lubricating property. Above all, products containing a low viscosity silicone oil have small sticky feeling, good spreadability and good extendability, and has high safety to human body, as compared with the conventional oily type products. For this reason, the silicone oil is continuing to be used in cosmetics, medical products, quasi drugs, and the like, on the basis of its characteristics. However, the conventional gelling agent is unable to sufficiently utilize the characteristics of the silicone oil, and gel strength is insufficient. In many cases, gelled products formed did not have cohesive force and were flaky, and dry feeling remained. In the case of using hydrophobic silica, bentonite and the like as an inorganic filler, the gelled product lacks in transparency, and has small spreadability. Additionally, because volatilization does not substantially proceed, sticky feeling remains over a long period of time. Thus, there was the problem that the advantages of a silicone oil are not sufficiently utilized.

Of gelled products using the conventional gelling agent, for example, the gelled product of Patent Document 1 has the problems that organomodified clay minerals become massed together, thereby a silicone oil is easy to separate. Furthermore, when the gel-like silicone oil composition is applied, sticky feeling develops. Patent Document 2 describes a gelled product of a silicone oil using fatty acid ester of sucrose. However, there is the problem that the gelled product loses refresh feeling inherent in a silicone oil. Patent Document 3 describes a gelling agent using a silicone oil having saturated hydrocarbon groups at both terminals. However, a production method of the gelling agent involves living polymerization of ethylene, and as a result, severe production control is required.

Patent Document 4 and Patent Document 5 describe a gelling agent using an oily dispersant of titanium oxide. However, it is considered that there is a problem in uniformity of a gelling agent. Patent Document 6 discloses a thickening and gelling agent for an oily substance, comprising a carboxylate of alkoxytitanium and a silicone oil having an amino group or a carboxyl group. However, when the gel obtained is applied to a skin, it provides poor spreadability and dry feeling. Thus, the gel is not sufficient to be used as cosmetics, medical products and quasi drugs.

Patent Document 1: JP-A 61-113646 (1986)
Patent Document 2: JP-A 63-235366 (1988)
Patent Document 3: JP-A 8-73744 (1996)
Patent Document 4: JP-A 2001-200160
Patent Document 5: JP-A 2002-212427
Patent Document 6: JP-A 2007-197646

### Disclosure of the Invention

### Problems that the Invention is to Solve

As described above, development of a thickening and gelling agent which is easily dispersed in an oily substance in a small addition amount, is free of liquid separation, stably forms a thickened material or a gel state, and can prepare a thickened product or a gel providing spreadability and free of sticky feeling and dry feeling has been demanded.

Particularly, a thickening and gelling agent regarding a silicone oil has not been put into practical use yet. Thus, development of a thickening and gelling agent having the above performances has been much-needed from fields of cosmetics, medical products and quasi drugs.

### Means for Solving the Problems

As a result of researches and investigations to solve the above problems, the present inventors have found that when (A) a modified product of alkoxytitanium or alkoxyzirconium with carboxylic acid and hydroxy compound, and a silicone oil having a functional group are combined, and the resulting mixture is used as a thickening and gelling agent, the thickening and gelling agent can widely thicken or gelatinize an oily substance, and a thickened product or a gelled product of an oily substance, having excellent properties of having spreadability, free of sticky feeling and dry feeling, and being smooth can be prepared. The present invention has been completed based on this finding.

The present invention provides a thickening and gelling agent for an oily substance, comprising:
(A) a modified product of alkoxytitanium or alkoxyzirconium with a carboxylic acid and a hydroxy compound, and
(B) at least one silicone oil selected from a silicone oil having an amino group and a silicone oil having a carboxyl group.

The present invention further provides an oily composition or a cosmetic preparation, comprising the thickening and gelling agent for an oily substance.

The present invention further provides a partial constituent for a thickening and gelling agent, comprising an oil solution having contained therein a modified product of alkoxytitanium or alkoxyzirconium with a carboxylic acid and a hydroxy compound, and a partial constituent for a thickening and gelling agent, comprising an oil solution having contained therein at least one selected from a silicone oil having an amino group and a silicone oil having a carboxyl group.

The present invention further provides a method for thickening and gelatinizing an oily substance, using the component (A) and the component (B) above.

### Advantage of the Invention

The thickening and gelling agent for an oily substance according to the present invention is easily dispersed in a wide range of oily substances including a silicone oil in a small addition amount thereof, and has the effects of prevention of flowing, shape retention, viscosity control, prevention of separation, and the like. The gelled product thus prepared is a thickened or gelled material having spreadability and free of sticky feeling, dry feeling, coloration and turbidity. The gelled product can be used in wide fields of paints, putty, adhesives, pharmaceutical preparations, inks, lubricants, cosmetics and foods.

### Best Mode for Carrying Out the Invention

A modified product of alkoxytitanium or alkoxyzirconium with a carboxylic acid and a hydroxy compound (the modified product is hereinafter referred to as "component (A)") used in the thickening and gelling agent for an oily substance of the present invention is produced by using alkoxytitanium or alkoxyzirconium as a raw material, and modifying the raw material with a carboxylic acid and a hydroxy compound. The alkoxytitanium or alkoxyzirconium used herein means not only tetraalkoxytitanium or tetraalkoxyzirconium as a monomer, but condensation polymers of those.

One example of the representative structural formula of the alkoxytitanium or alkoxyzirconium is represented by the following formula (I): 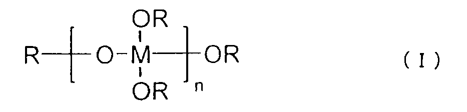 wherein at least one R represents a hydrocarbon group, the remainder represents a hydrocarbon group or a hydrogen atom, M represents titanium or a zirconium, and n is an integer of 1 or more.

In the above formula (I), the preferred hydrocarbon group (-R) is an alkyl group and an alkenyl group. In more specifically, examples of the hydrocarbon group include isopropyl group, n-propyl group, n-butyl group, tert-butyl group, isobutyl group, ethyl group, isooctyl group, stearyl group, isostearyl group and oleyl group. Alkoxytitanium and alkoxyzirconium, comprising mixed alkyl groups each having different hydrocarbon group are included. Of course, the hydrocarbon group is not limited to the groups exemplified above. In the above formula (I), in the case of n=1, the compound is a monomer, and in the case of n=2 or more, the compound is a condensation polymer.

Specific examples of the alkoxytitanium include tetraethoxytitanium, tetraisopropoxytitanium, tetra n-propoxytitanium, tetra n-butoxytitanium, tetra tert-butoxytitanium, tetraisobutoxytitanium, tetraoctoxytitanium, tetraisostearoxytitanium, and their condensation polymers. Specific examples of the alkoxyzirconium include tetraisopropoxyzirconium, tetra n-propoxyzirconium, tetra n-butoxyzirconium, tetra tert-butoxyzirconium, tetraisobutoxyzirconium, tetraoctoxyzirconium, and their condensation polymers. The formula (I) is exemplified as the representative structure, but actually, some compounds have further complicated structure, and it is considered that compounds in which a part of alkoxy groups is a hydroxyl group may be present.

Of the above alkoxytitanium, a tetraalkoxytitanium condensation polymer is industrially produced, and is sold by DuPont U.S.A., Matsumoto Fine Chemical Co., Ltd. , Nippon Soda Co., Ltd., and the like, and such a compound may be used.

Examples of the carboxylic acid used for the modification of the alkoxytitanium or the alkoxyzirconium include caproic acid, enanthic acid, caprylic acid, 2-ethylhexanoic acid, pelargonic acid, capric acid, undecylic acid, lauric acid, lauric acid, tridecylic acid, myristic acid, pentadecylic acid, palmitic acid, margaric acid, stearic acid, nondecylic acid, arahinic acid, lignosenic acid, isostearic acid, isooctanic acid, isopalmitic acid, behenic acid, 12-hydroxystearic acid, oleic acid, linoleic acid, linoleinic acid, recinoleic acid, adipic acid, decanedicarboxylic acid, benzoic acid, toluic acid, malic acid and tartaric acid. Of those, aliphatic carboxylic acids having a carbon number of 8 or more are particularly preferred.

Those carboxylic acids react with an alkoxy group of the alkoxytitanium or alkoxyzirconium to form titanium or zirconium carboxylate, thereby producing monohydric alcohol as by-product.

Examples of the hydroxy compound that can be used for the modification of alkoxytitanium or alkoxyzirconium include an ether compound having a hydroxyl group, an ester compound having a hydroxyl group, an amino compound having a hydroxyl group, an amide compound having a hydroxyl group, and a polyhydric alcohol. Of those, examples of the preferred compounds include a compound having a hydroxyl group, an ether bond and an ester bond in the same molecule, a compound having a hydroxyl group, an ether bond and an amino group in the same molecule, a compound having a hydroxyl group, an ester bond and an amino group in the same molecule, and a compound having a hydroxyl group, an amide bond and an ester bond in the same molecule, and those compounds can be used.

More specifically, examples of the ether compound having a hydroxyl group that can be used in a reaction include monoethers of glycol, such as ethylene glycol monobutyl ether, ethylene glycol monolauryl ether, ethylene glycol monooctyl ether, ethylene glycol monostearyl ether, ethylene glycol monobenzyl ether, propylene glycol monodecyl ether, propylene glycol monostearyl ether and hexylene glycol monostearyl ether; mono-and diethers of trihydric or more aliphatic alcohols, such as trimethylolethane monoisostearyl ether and trimethylolethane diisostearyl ether; polyalkylene glycols such as diethylene glycol, triethylene glycol, polyethylene glycol, dipropylene glycol, tripropylene glycol, polypropylene glycol and polyethylene propylene glycol; monoethers of polyalkylene glycol, such as diethylene glycol monobutyl ether, dipropylene glycol monononyl ether, polyoxyethylene lauryl ether, polyoxyethylene octyl phenyl ether, polyoxyethylene cetyl ether and polyoxyethylene/polyoxypropylene decyl ether; mono- and diethers of glycerin and alcohol, such as batyl alcohol, chimyl alcohol and glyceryl monoisostearyl ether; and polyglycerins such as diglycerin, triglycerin, tetraglycerin and decaglycerin.

Examples of the ester compound having a hydroxyl group that can be used in the reaction include monoesters of glycol, such as 2-hydroxyethyl acetate, 2-hydroxyethyl butyrate, 2-hydroxyethyl laurate, 2-hydroxyethyl caprate, 2-hydroxyethyl isostearate, 2-hydroxyethyl palmitate, 2-hydroxypropyl acetate, 2-hydroxypropyl butyrate, 2-hydroxypropyl laurate, 2-hydroxypropyl caprylate, 2-hydroxypropyl isostearate, 2-hydroxypropyl palmitate, 3-hydroxypropyl acetate, 3-hydroxypropyl butyrate, 3-hydroxypropyl laurate, 3-hydroxypropyl caprylate, 3-hydroxypropyl isostearate, 3-hydroxypropyl palmitate, 4-hydroxybutyl acetate, 4-hydroxybutyl butyrate, 4-hydroxybutyl laurate, 4-hydroxybutyl caprate, 4-hydroxybutyl isostearate and 4-hydroxybutyl palmitate; esters of trihydric or more aliphatic alcohols, such as trimethylolpropane monoisostearate, trimethylolpropane diisostearate, pentaerythritol monoisostearate, pentaerythritol 2-ethylhexanoate, glyceryl lactate, glyceryl isostearate, glyceryl diisostearate, glyceryl oleate, glyceryl dioleate, glyceryl palmitate and glyceryl dipalmitate; esters of saccharides, such as sorbitan monooleate and sorbitan monolaurate; and esters of oxycarboxylic acid and alcohol, such as cetyl lactate, lauryl lactate, myristyl lactate, octyldecyl lactate, diisostearyl malate, dimyristyl tartrate, trioctyl citrate, triethyl citrate, triisocetyl citrate and trioctyldecyl citrate. The ester compound further includes compounds having two bonds of an ester bond and an ether bond, other than a hydroxyl group, such as batyl monoisostearate, polyoxyethylene glyceryl stearate, polyoxyethylene sorbitan isostearate, polyethylene glycol myristate, polyethylene glycol laurate, polyglyceryl distearate and diglyceryl oleate.

Examples of the amino compound having a hydroxyl group that can be used in the reaction include N,N-dimethyl ethanolamine, N,N-diethyl ethanolamine, N,N-dibutyl ethanolamine, N,N-(β-aminoethyl) ethanolamine, N-methyl ethanolamine, N-methyl diethanolamine, monoethanolamine, diethanolamine, triethanolamine, N-ethyl ethanolamine, N-ethyl diethanolamine, N-butyl ethanolamine, N-butyl diethanolamine, N-tert-butyl diethanolamine, monoisopropanolamine, diisopropanolamine, triisopropanolamine, N-(β-aminoethyl) isopropapnolamine, N,N-diethyl isopropanolamine, stearamide ethyl ethanolamine and hydroxyethyl isostearoxyisopropanolamine. The amino compound further includes compounds having an ether bond and an amino group, other than a hydroxyl group, such as polyoxyethylene alkyl amine.

Examples of the amide compound having a hydroxyl group that can be used in the reaction include N,N-diethanol dodecanoic acid amide, N,N-diethanol hexadecanoic acid amide, N,N-diethanol linoleic acid amide, N,N-diethanol oleic acid amide, N-ethanol-dodecanoic acid amide, N-ethanol-N-methyl hexadecanoic acid amide, N-ethanol-linoleic acid amide and N-ethanol-N-methyl oleic acid amide, that can be prepared by reacting an alkanol amine and a fatty acid. The amide compound further includes N-octyl-3-hydroxypentadecanoic acid amide, N-dodecyl-6-hydroxyheptadecanoic acid amide, N-vehenyl-10-hydroxytridecanoic acid amide and N-hexadecyl-12-hydroxystearic acid amide, that can be prepared by reacting a fatty acid having a hydroxyl group and an amine. The amide compound further includes compounds having an amide bond and an ether bond, other than a hydroxyl group, obtained by adding alkylene oxide to those amide compounds having a hydroxyl group.

Examples of the polyhydric alcohol that can be used in the reaction include dihydric aliphatic alcohols such as ethylene glycol, 1,3-propanediol, 1,3-butanediol, 1,4-butanediol, 1,5-pentanediol, 1,4-pentanediol, 1,3-pentanediol, 3-methyl-1,5-pentanediol, 1,6-hexanediol, 1-methyl-1,8-octanediol, 2-ethyl-1,3-hexanediol, 2-methyl-2,4-pentanediol, 3-propyl-1,5-pentanediol, 3-methyl-1,6-hexanediol, 4-methyl-1,7-heptanediol, 4-methyl-1,8-octanediol, 4-propyl-1,8-octanediol and 1,9-nonanediol; trihydric or more aliphatic alcohols such as trimethylolethane, trimethylolpropene, glycerin and pentaerythritol; alicyclic alcohols such as 1,3-cyclohexane dimethanol, 1,2-cyclohexane dimethanol and hydrogenated bisphenols; polyvalent oxycarboxylic acids having a hydroxyl group, such as tartaric acid and citric acid; saccharides such as ribose, arabinose, glucose, mannose, galactose, fructose, saccharose and sorbitol; and castor oil.

A model of a production reaction of the component (A) of the present invention is described below. In the reaction formula below, one carboxylic acid residue (-CO-R'-) and one hydroxy compound residue (R"-O-) are introduced into the component (A), but actually, those residues are not limited to one, and can have various numeral values of 4 or less and 0.01 or more, depending on the conditions. 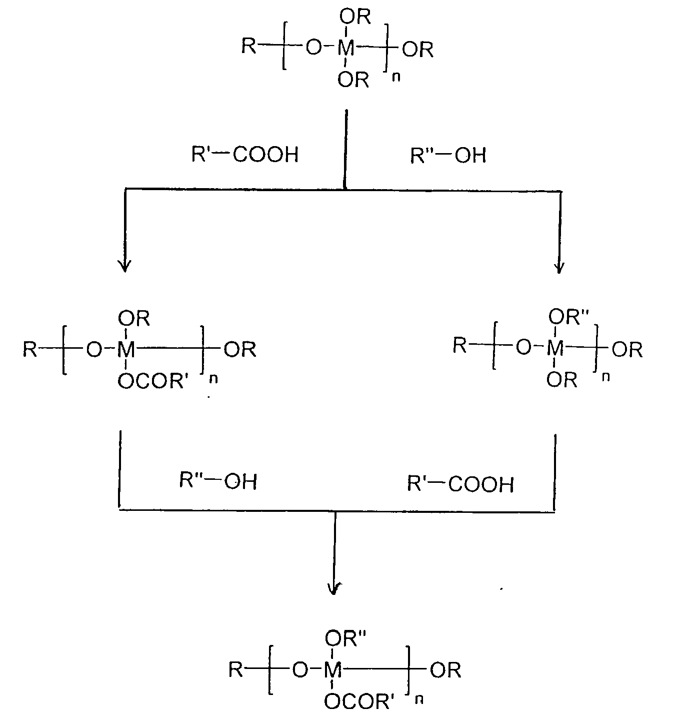 wherein R, M and n have the same meanings as defined before, R' represents a hydrocarbon group which may have a hydroxyl group, and R" represents a hydrocarbon group which may have a functional group.

The above reaction can easily be performed by mixing a carboxylic acid and a hydroxy compound with the alkoxytitanium or alkoxyzirconium (monomer or condensation polymer) at room temperature or under heating. The order of mixing the carboxylic acid and the hydroxy compound is not particularly limited. As a first step, the carboxylic acid may be reacted, and the hydroxy compound may then be reacted, or the reverse order may be employed. Furthermore, those compounds can be reacted simultaneously. Reaction temperature is preferably from room temperature to about 120°C.

The hydroxy compound performs an alcohol exchange reaction with an alkoxy group of the alkoxytitanium or alkoxyzirconium, thereby by-producing a monohydric alcohol. Therefore, it is preferred that the by-product alcohol is removed and the reaction is completed. In this case, the carboxylic acid and hydroxy compound can of course be used alone or 2 or more as mixtures thereof, respectively.

Amount of the carboxylic acid used in the above reaction is particularly preferably the number of molecules of from 0.2 to 4.0 times the total number of atoms of titanium and zirconium. Amount of the hydroxy compound used in the reaction is particularly preferably the number of molecules of from 0.02 to 3.0 times the total number of atoms of titanium and zirconium.

If necessary, alkoxytitanium derivatives and/or alkoxyzirconium derivatives modified by the above reaction can further be modified by adding water in the presence or absence of a solvent. By the additional modification step, a part of alkoxy groups is hydrolyzed to form a hydroxyl group, and may further be condensed and polymerized. Thereafter, the by-product alcohol, the solvent and unreacted water are preferably removed.

Materials preferably used as a solvent in the above additional modification step are hydrophilic alcohols, hydrophilic ethers, hydrophilic ketones and the like. Particularly preferred solvent is alcohols having a carbon number of from 2 to 4, and examples thereof include ethanol, isopropanol, n-propanol, n-butanol, isobutanol and tert-butanol. Alcohols having a carbon number of 5 or more are not preferred for the reason that solubility of water is decreased. Examples of the hydrophilic ethers include tetrahydroxyfuran and dioxane, and examples of the hydrophilic ketones include acetone and methyl ethyl ketone.

The compound (A) of the present invention thus obtained is combined with at least one silicone oil selected from a silicone oil having an amino group and a silicone oil having a carboxyl group, as the component (B). Thus, a thickening and gelling agent for an oily substance is obtained.

The silicone oil having an amino group or the silicone oil having a carboxyl group, as the component (B) means a silicone oil containing at least one amino group or carboxyl group in one molecule, respectively, and is obtained by mainly introducing an organic group having the respective amino group or carboxyl group into a side chain or terminals of a dimethyl silicone oil or a methyl phenyl silicone oil. Kind of the component (B) is not particularly limited. Representative examples of the component (B) are compounds represented by the following formula (II) and formula (III):

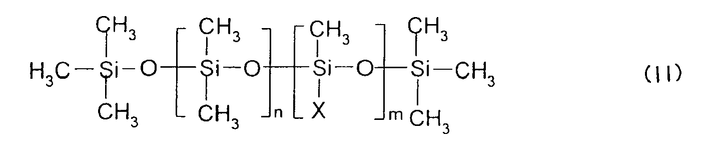

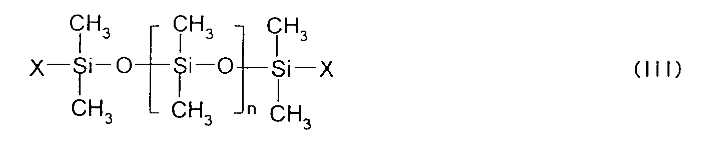

wherein X represents an organic group having a carboxyl group or an organic group having an amino group, n is an integer of from 10 to 10,000, and m is an integer of from 1 to 1,000.

In the above formulae, examples of the organic group having an amino group include -C₂H₄NH₂ and -C₃H₆NHCH₂NH₂, and example of the organic group having a carboxyl group includes -C₂H₄COOH.

Of the component (B), the silicone oil having an amino group sold by silicone makers can be used. The products include SF8452C (aminoethylaminopropylmethicone/dimethicone) copolymer, SS-3511 (aminoethylaminopropylmethicone/dimethicone) copolymer, SS-3552 (aminoethylaminopropylmethicone/dimethicone) copolymer and SF8457C (aminoethylaminopropylmethicone/dimethicone) copolymer, sold by Dow Corning Toray Co., Ltd.; KF-8010, X-22-161A, KF-860, KF-393, KF-859, KF-861, KF-867, KF-869, KF-880, KF-8002, KF-8004, KF-8005, KF-858, KF-864, KF-865, KF-868 and KF-8003, sold by Shin-Etsu Silicone Co., Ltd.; and TSF405-15K, XF42-B1989, XF42-B8922, TSF4702, TSF4703, TSF4704, TSF4705, TSF4706, TSF4707 and TSF4709, sold by Momentive Performance Materials, Japan.

The silicone oil having an amino group includes silicone oils having other organic group besides an amino group, and such silicone oils can be used. Examples of the silicone oil having a polyether group other than an amino group include X-22-3939A and X-22-3908A, sold by Shin-Etsu Silicone Co., Ltd., and examples of the silicone oil having an alkoxy group other than an amino group include XF-857, KF-862 and KF-8001, sold by Shin-Etsu Silicone Co., Ltd.

Similarly, of the component (B), silicone oils sold by silicone makers can be used as the silicone oil having a carboxyl group. Examples of the silicone oil include BY16-880 and SF8418, sold by Dow Corning Toray Co., Ltd.; X-22-162C, X-22-3701E and X-22-3710, sold by Shin-Etsu Silicone Co., Ltd.; and TSF4770, sold by Momentive Performance Materials, Japan.

The thickening and gelling agent of the present invention is used by combining the component (A) and the component (B). Mixing ratio between the component (A) and the component (B) in this case is particularly preferably from 100:10 to 1:100, in weight ratio.

In the case that the component (A) and the component (B) of the present invention are directly mixed, hard gel state is formed, and even though an oily substance is subsequently added, it is difficult to form a uniform state. In order to make the state uniform, heating and prolonged stirring may be required, and this is not practical. Consequently, in practical use, it is preferred to prepare a composition for a thickening and gelling agent comprising an oil solution described hereinafter having contained therein the component (A) and the component (B), a partial constituent for a thickening and gelling agent, comprising an oil solution having contained therein the component (A), or a partial constituent for a thickening and gelling agent, comprising an oil solution having contained therein the component (B).

The thickening and gelling agent of the present invention thus produced can effectively gelatinize or thicken the oily substance. The oily substance capable of being thickened and gelatinized is a liquid or semi-solid substance having relatively low polarity, and examples thereof include mineral oils, vegetable oils, animal oils, higher fatty acids, higher alcohols, higher esters, silicone oils and liquid resins. Of the specific oily substances, examples of the mineral oils include liquid paraffin, isoparaffin, kerosene, mineral sprit, xylene, toluene and vaseline. Examples of the vegetable oils include castor oil, avocado oil, olive oil, macadamia nut oil, safflower oil, sesame oil, soybean oil, camellia oil, cottonseed oil, rapeseed oil and almond oil. Examples of the animal oils include egg yolk oil, torta oil, horse oil, fish oil and lanolin. Examples of the higher fatty acids include behenic acid, arachidonic acid, isostearic acid, undecylic acid, oleic acid and soft lanolinic acid. Examples of the higher alcohols include isostearyl alcohol, oleyl alcohol, octyldodecyl alcohol and hexyldecyl alcohol. Examples of the higher esters include isocetyl isostearate, cetyl octanoate, cetostearyl octanoate, decyl oleate, isocetyl stearate, isopropyl myristate, octyldodecyl myristate, hexyl laurate and propylene glycol dicaprate. Examples of the glyceryl esters include glyceryl tri 2-ethylhexanoate, glyceryl trilaurate, glyceryl tripalminate, glyceryl tristearate, glyceryl trioleate, glyceryl tribehenate, cetyl lactate and diisostearyl malate. Examples of the silicone oils include dimethyl polysiloxane, methylphenyl polysiloxane, octamethyl cyclotetrasiloxane, decamethyl cyclopentasiloxane and polyoxyethylene/methylpolysiloxane. Examples of the liquid resins include a liquid epoxy resin and a liquid butadiene resin.

A method for mixing the thickening and gelling agent of the present invention with the oily substance is not particularly limited. However, for the reason described before, the mixing is preferably conducted such that the component (A) and the component (B) are mixed together with the oily substance, rather than the embodiment that as a first step the component (A) and the component (B) are mixed and then the resulting mixture is mixed with the oily substance. Specifically, the method includes a method of mixing the composition for the thickening and gelling agent in which the component (A) and the component (B) are mixed with an oil solution of an amount sufficient to dissolve those, with an oily substance to be thickened and gelatinized, and a method of adding a partial constituent for a thickening and gelling agent in which the component (A) or the component (B) is mixed with an oil solution of an amount sufficient to dissolve those, to an oily substance to be thickened and gelatinized, and then further adding the component (B) or the component (A). The oil solution used herein includes the oily substance described above, and general organic solvents having polarity.

Mixing ratio between the thickening and gelling agent and the oily substance varies depending on intended viscosity characteristics, and is therefore not particularly restricted. Generally, the mixing ratio is preferably from 10:25 to 10:300 in weight ratio.

The method for thickening and gelatinizing an oily substance using the thickening and gelling agent of the present invention can be used in wide fields such as cosmetics, paints, putty, adhesives, pharmaceutical preparations, inks and lubricants. The method can thicken and gelatinize silicone oils conventionally considered to be difficult to thicken and gelatinize, thereby obtaining a gelled product having spreadability, and free of sticky feeling and dry feeling. Therefore, the method has large possibility of utilization in fields of cosmetic preparations, medical products and quasi drugs.

For example, to utilize the thickening and gelling agent of the present invention in cosmetics, the thickening and gelling agent of the present invention is mixed with an oily substance usable as cosmetics at any step in the production of cosmetics. In such a case, in addition to the above-described essential components, pigments, dyes, dispersants, preservatives, ultraviolet absorbers, antioxidants, perfumes, solvents, water, surfactants, antiperspirants, moisturizers, resins and the like can be used.

### Examples

The present invention is described in further detail below by reference to Examples and Comparative Examples, but it should be understood that the invention is not restricted by those Examples and the like.

### Example 1

14.2 g of isostearic acid was added to 14.2 g of tetraisopropoxytitanium, and the resulting mixture was gradually heated and maintained at 100 to 110°C for 30 minutes. The mixture was cooled to room temperature, 30.0 g of a polypropylene glycol having a molecular weight of 2,000 was added thereto, and the resulting mixture was again heated and maintained at 110 to 120°C for 30 minutes. Thereafter, volatile substances were removed at 90 to 95°C under reduced pressure of 10 mmHg. Thus, 53.5 g of pale yellow viscous liquid (S-1) of an alkoxytitanium derivative was obtained. The estimated chemical formula is Ti (COOSt) (OPr)_{2.4}(OC₃H₆O)_{0.3}. As a result of elemental analysis, titanium is 4.42%, carbon is 62. 50%, hydrogen is 10.77%, and oxygen is 22.31%. The theoretical values are 4.46%, 62.60%, 10.66% and 22.28%, respectively. In the chemical formula, St represents an isostearyl group, and Pr represents an isopropyl group.

### Examples 2 to 7

Dimethyl polysiloxane (TSF-451-5A; sold by Momentive Performance Materials, Japan) which is a linear silicone oil and dodecanemethyl cyclohexasiloxane (DC246; sold by Dow Corning Toray Co., Ltd.) which is a cyclic silicone oil, as an oily substance; kerosene, toluene, isononyl isonanoate and cetyl octanoate were added to the alkoxytitanium derivative S-1 prepared in Example 1, respectively, according to the compositions shown in Table 1. XF42-B8922 (sold by Momentive Performance Materials, Japan, viscosity: 70 Pa·s, amine equivalent: 13,000 g/mol) which is a silicone oil having an amino group and X-22-162C (sold by Shin-Etsu Silicone Co., Ltd., viscosity: 230 mm²/s, carboxyl equivalent: 2, 300 g/mol) which is a silicone oil having a carboxyl group were added to the respective compositions. Thus, the compositions of Examples 2 to 7 were prepared.

After mixing, each composition was allowed to stand at room temperature for 24 hours, and thickening and gelling properties, appearance, liquid separation, spreadability and smooth feeling were evaluated as follows. The results are shown in Table 1.

### <Thickening and gelling properties>

⊙: Gelled
○: Thickened
×: Not thickening

### <Appearance>

⊙: Transparent appearance
○: Slightly turbid appearance
×: Turbid appearance

### <Liquid separation>

⊙: No liquid separation
×: Liquid separation is seen

### <Spreadability>

⊙: In sensory evaluation by five persons, five persons obtained good spreadability and light feeling
○: In sensory evaluation by five persons, three or four persons obtained good spreadability and light feeling
△: In sensory evaluation by five persons, one or two persons obtained good spreadability and light feeling
×: In sensory evaluation by five persons, no one obtained good spreadability and light feeling
The sensory evaluation was evaluated by feeling when a small amount of a thickened and gelled product was applied to a skin and spread thereon.

### <Smooth feeling>

⊙: In sensory evaluation by five persons, five persons obtained smooth feeling free of sticky feeling
○: In sensory evaluation by five persons, three or four persons obtained smooth feeling free of sticky feeling
△: In sensory evaluation by five persons, one or two persons obtained smooth feeling free of sticky feeling
×: In sensory evaluation by five persons, no one obtained smooth feeling free of sticky feeling
The sensory evaluation was evaluated by feeling after applying a small amount of a thickened and gelled product to a skin.

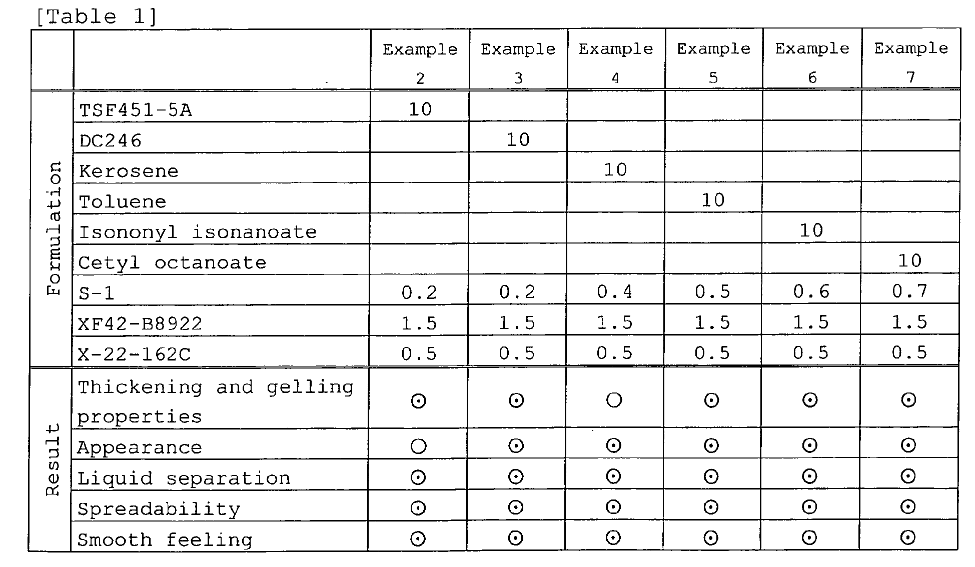

### Examples 8 to 13

Decamethyl cyclopentasiloxane (SH245; sold by Dow Corning Toray Co., Ltd.) which is a cyclic silicone oil was added as an oily substance to the alkoxytitanium derivative (S-1) prepared in Example 1 according to the compositions shown in Table 2, and a silicone oil having an amino group or a carboxyl group was then added thereto in an respective amount shown in Table 2. Thus, thickened and gelled products of Examples 8 to 13 were prepared. XF42-B8922 (sold by Momentive Performance Materials, Japan) was used as the silicone oil having an amino group. X-22-162C (sold by Shin-Etsu Silicone Co., Ltd.), X-22-3701E (sold by Shin-Etsu Silicone Co., Ltd., viscosity: 2,000 mm²/s, carboxyl equivalent: 4, 000 g/mol) and X-22-3710 (sold by Shin-Etsu Silicone Co., Ltd., viscosity: 55 mm²/s, carboxyl equivalent: 1,400 g/mol) were used as the silicone oil having a carboxyl group, respectively. Similar to the above Examples, the thickened and gelled products thus obtained were examined for thickening and gelling properties, appearance, liquid separation, spreadability and smooth feeling. The results are shown in Table 2.

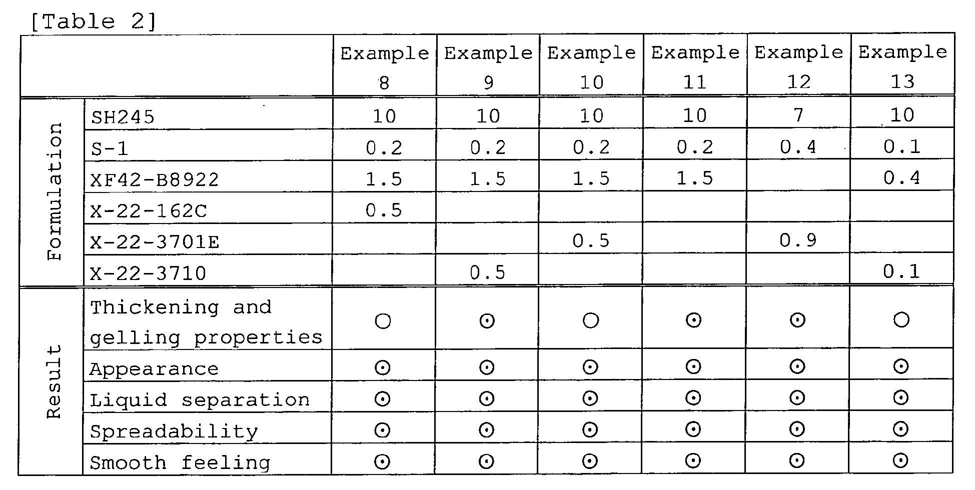

### Example 14

42.6 g of isostearic acid was added to 14.2 g of tetraisopropoxytitanium, and the resulting mixture was gradually heated and maintained at 80 to 85°C for 120 minutes. The mixture was cooled to room temperature, 10.0 g of a polypropylene glycol having a molecular weight of 1,000 was added thereto, and the resulting mixture was again heated and maintained at 110 to 120°C for 30 minutes. Thereafter, volatile substances were removed at 90 to 95°C under reduced pressure of 10 mmHg. Thus, 56.5 g of pale yellow viscous liquid (S-2) of an alkoxytitanium derivative was obtained. Titanium content of the estimated chemical formula of the liquid was 4.23%, and the measured value was 4.24%.

### Example 15

28.4 g of isostearic acid was added to 14.2 g of tetraisopropoxytitanium, and the resulting mixture was gradually heated and maintained at 80 to 85°C for 120 minutes. The mixture was cooled to room temperature, 15.0 g of a polyethylene glycol having a molecular weight of 200 was added thereto, and the resulting mixture was again heated and maintained at 110 to 120°C for 30 minutes. Thereafter, volatile substances were removed at 90 to 95°C under reduced pressure of 10 mmHg. Thus, 45.8 g of slight yellow viscous liquid (S-3) of an alkoxytitanium derivative was obtained. Titanium content of the estimated chemical formula of the liquid was 5.25%, and the measured value was 5.31%.

### Example 16

28.4 g of isostearic acid was added to 14.2 g of tetraisopropoxytitanium, and the resulting mixture was gradually heated and maintained at 80 to 85°C for 120 minutes. The mixture was cooled to room temperature, 17. 5 g of diglyceryl diisostearate was added thereto, and the resulting mixture was maintained at 100 to 120°C for 30 minutes. 1.9 g of 1, 3-propanediol was further added thereto, and the resulting mixture was maintained at 100 to 120°C for 30 minutes. Thereafter, volatile substances were removed at 90 to 95°C under reduced pressure of 10 mmHg. Thus, 51.2 g of pale yellow viscous liquid (S-4) of an alkoxytitanium derivative was obtained. Titanium content of the estimated chemical formula of the liquid was 4.78%, and the measured value was 4.70%.

### Example 17

20.0 g of a polypropylene glycol having a molecular weight of 2,000 was added to 14.2 g of tetraisopropoxytitanium, and the resulting mixture was gradually heated and maintained at 100 to 110°C for 30 minutes. The mixture was cooled to room temperature, 56.8 g of isostearic acid was added thereto, and the resulting mixture was again heated and maintained at 110 to 120°C for 30 minutes. Heating was further continued, and volatile substances were removed at 90 to 95°C under reduced pressure of 10 mmHg. Thus, 79.1 g of slight yellow viscous liquid (S-5) containing an alkoxytitanium derivative was obtained. Titanium content of the estimated chemical formula of the liquid was 3.03%, and the measured value was 3.03%.

### Example 18

42.6 g of isostearic acid was added to 14.2 g of tetraisopropoxytitanium, and the resulting mixture was gradually heated and maintained at 100 to 110°C for 30 minutes. The mixture was cooled to room temperature, 20.0 g of a polypropylene glycol having a molecular weight of 2,000 was added thereto, and the resulting mixture was again heated and maintained at 110 to 120°C for 30 minutes. 1.8 g of water was further added thereto, and heating was continued for 30 minutes. Thereafter, volatile substances were removed at 90 to 95°C under reduced pressure of 10 mmHg. Thus, 64.9 g of pale yellow viscous liquid (S-6) of an alkoxytitanium derivative was obtained. Titanium content of the estimated chemical formula of the liquid was 3.66%, and the measured value was 3.60%.

### Example 19

28.4 g of isostearic acid was added to 14.2 g of tetraisopropoxytitanium, and the resulting mixture was gradually heated and maintained at 100 to 110°C for 30 minutes. The mixture was cooled to room temperature, 21.6 g of diglyceryl monoisostearate was added thereto, and the resulting mixture was again heated and maintained at 110 to 120°C for 30 minutes. Thereafter, volatile substances were removed at 90 to 95°C under reduced pressure of 10 mmHg. Thus, 54.5 g of pale yellow viscous liquid (S-7) of an alkoxytitanium derivative was obtained. Titanium content of the estimated chemical formula of the liquid was 4.58%, and the measured value was 4.38%.

### Example 20

28.4 g of isostearic acid was added to 14.2 g of tetraisopropoxytitanium, and the resulting mixture was gradually heated and maintained at 100 to 110°C for 30 minutes. The mixture was cooled to room temperature, 24.1 g of diglyceryl triisostearate was added thereto, and the resulting mixture was maintained at 100 to 120°C for 30 minutes. Thereafter, volatile substances were removed at 90 to 95°C under reduced pressure of 10 mmHg. Thus, 58.8 g of pale yellow viscous liquid (S-8) of an alkoxytitanium derivative was obtained. Titanium content of the estimated chemical formula of the liquid was 4.04%, and the measured value was 4.05%.

### Example 21

42.6 g of isostearic acid was added to 14.2 g of tetraisopropoxytitanium, and the resulting mixture was gradually heated and maintained at 100 to 110°C for 30 minutes. The mixture was cooled to room temperature, 9.3 g of castor oil was added thereto, and the resulting mixture was again heated and maintained at 110 to 120°C for 30 minutes. Thereafter, volatile substances were removed at 90 to 95°C under reduced pressure of 10 mmHg. Thus, 56.2 g of pale yellow viscous liquid (S-9) of an alkoxytitanium derivative was obtained. Titanium content of the estimated chemical formula of the liquid was 4.32%, and the measured value was 4.25%.

### Example 22

42.6 g of isostearic acid was added to 14.2 g of tetraisopropoxytitanium, and the resulting mixture was gradually heated and maintained at 80 to 85°C for 120 minutes. The mixture was cooled to room temperature, 7.5 g of triethanolamine was added thereto, and the resulting mixture was again heated and maintained at 110 to 120°C for 30 minutes. Thereafter, volatile substances were removed at 90 to 95°C under reduced pressure of 10 mmHg. Thus, 52.4 g of yellow viscous liquid (S-10) of an alkoxytitanium derivative was obtained. Titanium content of the estimated chemical formula of the liquid was 4.58%, and the measured value was 4.55%.

### Example 23

42.6 g of isostearic acid was added to 14.2 g of tetraisopropoxytitanium, and the resulting mixture was gradually heated and maintained at 100 to 110°C for 30 minutes. The mixture was cooled to room temperature, 8.0 g of diisostearyl malate was added thereto, and the resulting mixture was again heated and maintained at 110 to 120°C for 30 minutes. Thereafter, volatile substances were removed at 90 to 95°C under reduced pressure of 10 mmHg. Thus, 55.0 g of pale yellow viscous liquid (S-11) of an alkoxytitanium derivative was obtained. Titanium content of the estimated chemical formula of the product was 4.30%, and the measured value was 4.35%.

### Example 24

28.4 g of isostearic acid was added to 14.2 g of tetraisopropoxytitanium, and the resulting mixture was gradually heated and maintained at 100 to 110°C for 30 minutes. The mixture was cooled to room temperature, 9.0 g of 1, 3-propanediol was added thereto, and the resulting mixture was again heated and maintained at 110 to 120°C for 30 minutes. Thereafter, volatile substances were removed at 90 to 95°C under reduced pressure of 10 mmHg. Thus, 40.0 g of pale yellow liquid of an alkoxytitanium derivative was obtained. This was slight yellow viscous liquid (S-12). Titanium content of the estimated chemical formula of the liquid was 6.0%, and the measured value was 6.00%.

### Example 25

42.6 g of isostearic acid was added to 14.2 g of tetraisopropoxytitanium, and the resulting mixture was gradually heated and maintained at 100 to 110°C for 30 minutes. The mixture was cooled to room temperature, 20 g of a polypropylene glycol having a molecular weight of 2,000 was added thereto, and the resulting mixture was maintained at 100 to 120°C for 30 minutes. 8.9 g of monoglyceryl isostearate was further added thereto, and the resulting mixture was maintained at 100 to 120°C for 30 minutes. Thereafter, volatile substances were removed at 90 to 95°C under reduced pressure of 10 mmHg. Thus, 73.8 g of pale yellow viscous liquid (S-13) of an alkoxytitanium derivative was obtained. Titanium content of the estimated chemical formula of the liquid was 3.23%, and the measured value was 3.23%.

### Example 26

28.4 g of isostearic acid was added to 14.2 g of tetraisopropoxytitanium, and the resulting mixture was gradually heated and maintained at 100 to 110°C for 30 minutes. The mixture was cooled to room temperature, 12.2 g of N-ethanol-lauric acid amide was added thereto, and the resulting mixture was maintained at 100 to 120°C for 30 minutes. Thereafter, volatile substances were removed at 90 to 95°C under reduced pressure of 10 mmHg. Thus, 45.8 g of pale yellow viscous liquid (S-14) of an alkoxytitanium derivative was obtained. Titanium content of the estimated chemical formula of the liquid was 5.23%, and the measured value was 5.33%.

### Examples 27 to 39

Decamethyl cyclopentasiloxane (SH245; sold by Dow Corning Toray Co., Ltd.) which is a cyclic silicone oil was added as an oily substance to XF42-B8922 (sold by Momentive Performance Materials, Japan) which is a silicone oil having an amino group and X-22-162C (sold by Shin-Etsu Silicone Co., Ltd.) which is a silicone oil having a carboxyl group according to the compositions of Table 3. Subsequently, alkoxytitanium derivatives (S-2 to S-14) modified with various hydroxy compounds, that is, an ether compound having a hydroxyl group, an ester compound having a hydroxyl group, an amino compound having a hydroxyl group, an amide compound having a hydroxyl group and a polyhydric alcohol, were added thereto. Thus, thickened and gelled products were prepared. The thickened and gelled products thus obtained were examined for their performances, that is, thickening and gelling properties, appearance, liquid separation, spreadability and smooth feeling. The results are shown in Table 3.

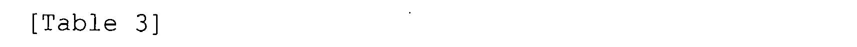

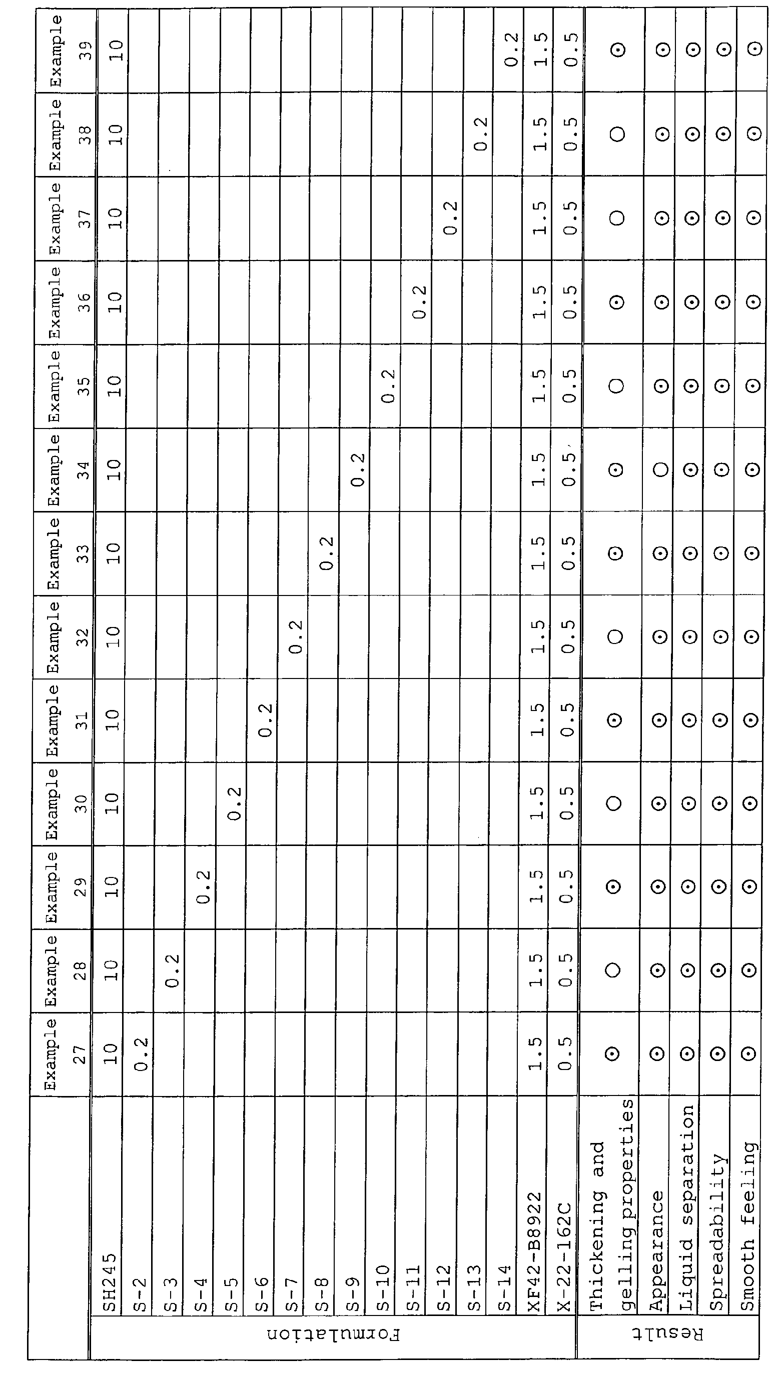

### Example 40

21.3 g of isostearic acid was added to 13.8 g of a dimer of tetra n-butoxytitanium, and the resulting mixture was gradually heated and maintained at 100 to 110°C for 30 minutes. The mixture was cooled to room temperature, 30 g of a polypropylene glycol having a molecular weight of 2,000 was added thereto, and the resulting mixture was again heated and maintained at 110 to 120°C for 30 minutes. Thereafter, volatile substances were removed at 90 to 95°C under reduced pressure of 10 mmHg. Thus, 57.0 g of pale yellow liquid (S-15) of an alkoxytitanium derivative was obtained. This was slight yellow viscous liquid. Titanium content of the estimated chemical formula of the liquid was 4.05%, and the measured value was 4.20%.

### Example 41

30.0 g of lauric acid was added to 14.2 g of tetraisopropoxytitanium, and the resulting mixture was gradually heated and maintained at 100 to 110°C for 30 minutes. The mixture was cooled to room temperature, 20 g of a polypropylene glycol having a molecular weight of 2,000 was added thereto, and the resulting mixture was maintained at 100 to 120°C for 30 minutes. Thereafter, volatile substances were removed at 90 to 95°C under reduced pressure of 10 mmHg. Thus, 54.7 g of pale yellow wax-like solid (S-16) of an alkoxytitanium derivative was obtained. When this was heated to 80°C, pale yellow liquid was obtained. Titanium content of the estimated chemical formula of the liquid was 4.43%, and the measured value was 4.36%.

### Example 42

51.0 g of behenic acid was added to 14.2 g of tetraisopropoxytitanium, and the resulting mixture was gradually heated and maintained at 100 to 110°C for 30 minutes. The mixture was cooled to room temperature, 10 g of a polypropylene glycol having a molecular weight of 1,000 was added thereto, and the resulting mixture was maintained at 100 to 120°C for 30 minutes. Thereafter, volatile substances were removed at 90 to 95°C under reduced pressure of 10 mmHg. Thus, 65.7 g of pale yellow wax-like solid (S-17) of an alkoxytitanium derivative was obtained. When this was heated to 80°C, pale yellow liquid was obtained. Titanium content of the estimated chemical formula of the liquid was 3. 68%, and the measured value was 3.65%.

### Example 43

42.6 g of n-stearic acid was added to 22.5 g of 85% n-butanol solution of tetra n-butoxyzirconium, and the resulting mixture was gradually heated and maintained at 100 to 110°C for 30 minutes. The mixture was cooled to room temperature, 10 g of a polyethylene glycol having a molecular weight of 200 was added thereto, and the resulting mixture was maintained at 100 to 120°C for 30 minutes. Thereafter, volatile substances were removed at 90 to 95°C under reduced pressure of 10 mmHg. Thus, 57.1 g of wax-like solid (S-18) of an alkoxyzirconium derivative was obtained. When this was heated to 80°C, slight yellow liquid was obtained. Zirconium content of the estimated chemical formula of the liquid was 8.00%, and the measured value was 7.95%.

### Comparative Synthesis Example 1

14.2 g of isostearic acid was added to 14.2 g of tetraisopropoxytitanium, and the resulting mixture was gradually heated and maintained at 100 to 110°C for 30 minutes. Thereafter, volatile substances were removed at 90 to 95°C under reduced pressure of 10 mmHg. Thus, 25. 5 g of pale yellow viscous liquid (H-1) of an alkoxytitanium derivative was obtained. Titanium content of the estimated chemical formula of the liquid was 9.42%, and the measured value was 9.40%.

### Comparative Synthesis Example 2

42.6 g of isostearic acid was added to 14.2 g of tetraisopropoxytitanium, and the resulting mixture was gradually heated and maintained at 80 to 85°C for 120 minutes. Thereafter, volatile substances were removed at 90 to 95°C under reduced pressure of 10 mmHg. Thus, 48.0 g of pale yellow viscous liquid (H-2) of an alkoxytitanium derivative was obtained. Titanium content of the estimated chemical formula of the liquid was 5.00%, and the measured value was 5.00%.

### Examples 44 to 47 and Comparative Examples 1 to 2

Decamethyl cyclopentasiloxane (SH245; sold by Dow Corning Toray Co. , Ltd.) which is a cyclic silicone oil was added as an oily substance to each of the alkoxytitanium derivative (S-15) using a dimer of tetrabutoxytitanium, the alkoxytitanium derivative (S-16) using lauric acid, the alkoxytitanium derivative (S-17) using behenic acid and the alkoxyzirconium derivative (S-18) using tetra n-butoxyzirconium according to Table 4. Subsequently, XF42-B8922 (sold by Momentive Performance Materials, Japan) which is a silicone oil having an amino group and X-22-162C (sold by Shin-Etsu Chemical Co. , Ltd.) which is a silicone oil having a carboxyl group were added to each of the mixtures.

Thickened and gelled products obtained above were examined for their performances, that is, thickening and gelling properties, appearance, liquid separation, spreadability and smooth feeling. Additionally, the cases using titanium compounds H-1 and H-2 produced in Comparative Synthesis Example 1 and Comparative Synthesis Example 2, respectively, were examined as comparative examples. The results are shown in Table 4.

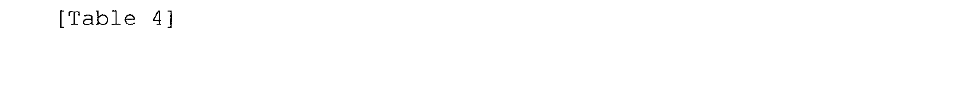

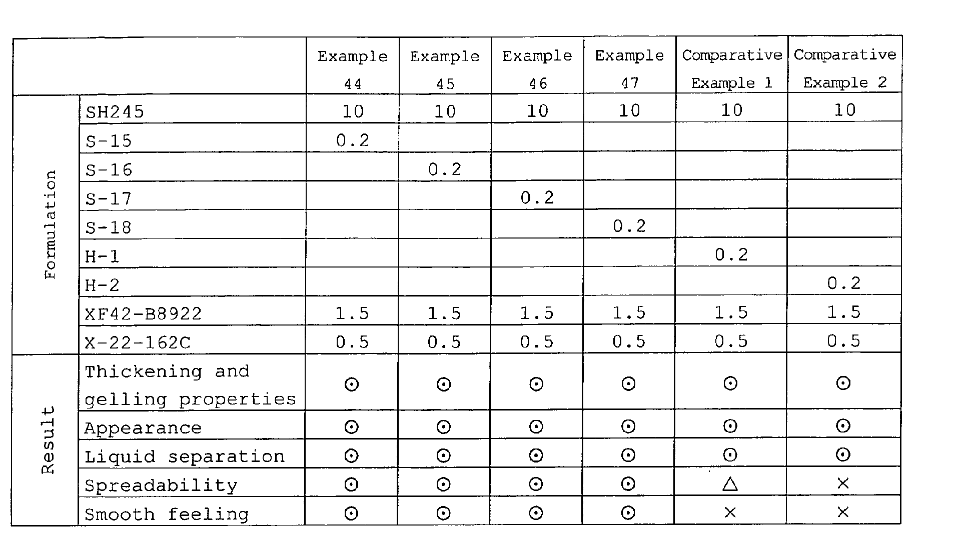

### Example 49

Of components shown in Table 5, as a first step, components B were mixed. A mixture of components C was added to the mixture of components B while stirring, and a material obtained by mixing and pulverizing components A was added to the resulting mixture while stirring. Thus, an oily foundation was produced. The alkoxytitanium derivative used is S-1 obtained in Example 1, the silicone oil having an amino group is XF42-B8922 (sold by Momentive Performance Materials, Japan), and the silicone oil having a carboxyl group is X-22-162C (sold by Shin-Etsu Silicone Co., Ltd.). Decamethyl cyclopentasiloxane (SH245, sold by Dow Corning Toray Co. , Ltd.) which is a cyclic silicone oil, liquid paraffin, octyl palmitate and vaseline were used as an oily substance. The oily foundation thus obtained was free of sticky feeling, and had excellent smooth feeling, spreadability and storage stability.

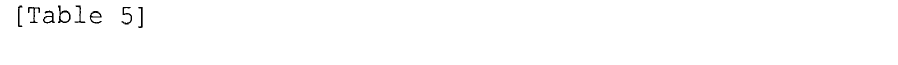

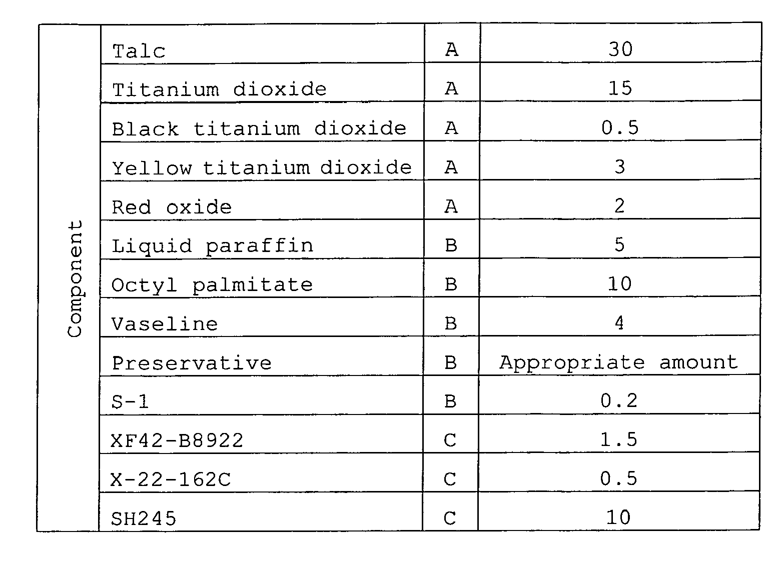

### Example 50

Of components shown in Table 6, components A were dispersed using a roller mill. Components C were heated and melted. Components B were mixed to prepare a mixed liquid, and components A, C and D were added to the mixed liquid. The resulting mixture was poured in a mold. Thus, an oily type lipstick was produced. S-1 obtained in Example 1 was used as the aloxytitanium derivative, XF42-B8922 (sold by Momentive Performance Materials, Japan) was used as the silicone oil having an amino group, and X-22-162C (sold by Shin-Etsu Silicone Co., Ltd.) was used as the silicone oil having a carboxyl group. Lanolin, liquid paraffin and decamethyl cyclopentasiloxane (SH245, sold by Dow Corning Toray Co. , Ltd.) which is a cyclic silicone oil were used as an oily substance. The lipstick thus obtained was free of sticky feeling, and had excellent smooth feeling, spreadability, storage stability and gloss.

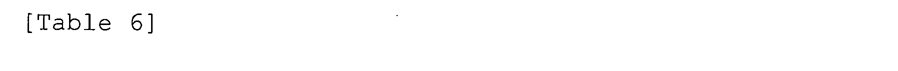

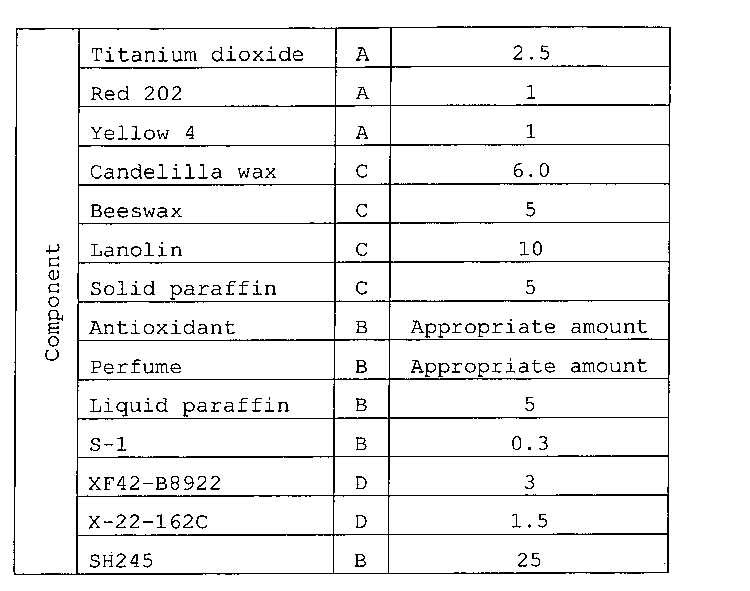

### Industrial Applicability

The thickening and gelling agent according to the present invention has the effects of prevention of flowing of an oily substance, shape retention, viscosity control, prevention of separation, and the like. It is possible that the thickening and gelling agent is utilized in wide fields of cosmetic preparations, paints, putty, adhesives, pharmaceutical preparations, inks, lubricants and the like. In particular, a silicone oil can be thickened and gelatinized, and the thickened and gelled product has spreadability and is free of sticky feeling and dry feeling. Therefore, the thickening and gelling agent can greatly contribute to fields of cosmetic preparations, medical products and quasi drugs.

## Claims

1. A thickening and gelling agent for an oily substance, comprising the following components (A) and (B):
(A) a modified product of alkoxytitanium or alkoxyzirconium with a carboxylic acid and a hydroxy compound, and
(B) at least one silicone oil selected from a silicone oil having an amino group and a silicone oil having a carboxyl group.

2. The thickening and gelling agent for an oily substance according to claim 1, wherein the hydroxy compound of the component (A) is at least one selected from an ether compound having a hydroxyl group, an ester compound having a hydroxyl group, an amino compound having a hydroxyl group, an amide compound having a hydroxyl group, and a polyhydric alcohol.

3. The thickening and gelling agent for an oily substance according to claim 1 or 2, which thickens and gelatinizes a silicone oil.

4. An oily composition comprising the thickening and gelling agent for an oily substance according to any one of claims 1 to 3.

5. A cosmetic preparation comprising the thickening and gelling agent for an oily substance according to any one of claims 1 to 3.

6. An oily composition comprising the thickening and gelling agent for an oily substance according to any one of claims 1 to 3, and a silicone oil.

7. A cosmetic preparation comprising the thickening and gelling agent for an oily substance according to any one of claims 1 to 3, and a silicone oil.

8. A partial constituent for a thickening and gelling agent, comprising an oil solution having contained therein a modified product of alkoxytitanium or alkoxyzirconium with a carboxylic acid and a hydroxy compound.

9. A partial constituent for a thickening and gelling agent, comprising an oil solution having contained therein at least one selected from a silicone oil having an amino group and a silicone oil having a carboxyl group.

10. A method for thickening and gelatinizing an oily substance, comprising the steps of:
adding one of the following components (A) and (B):
(A) a modified product of alkoxytitanium or alkoxyzirconium with a carboxylic acid and a hydroxy compound,
(B) at least one silicone oil selected from a silicone oil having an amino group and a silicone oil having a carboxyl group,
to an oil solution which may contain an oily substance to be gelatinized; and
adding the other component to the resulting mixture, followed by mixing.

11. A method for thickening and gelatinizing an oily substance, comprising the steps of:
adding one of the following components (A) and (B):
(A) a modified product of alkoxytitanium or alkoxyzirconium with a carboxylic acid and a hydroxy compound,
(B) at least one silicone oil selected from a silicone oil having an amino group and a silicone oil having a carboxyl group,
to an oil solution which does not contain an oily substance to be gelatinized; and
adding the other component and an oily substance to be gelatinized to the resulting mixture, followed by mixing.

12. A method for thickening and gelatinizing an oily substance, comprising the steps of:
adding the following components (A) and (B):
(A) a modified product of alkoxytitanium or alkoxyzirconium with a carboxylic acid and a hydroxy compound,
(B) at least one silicone oil selected from a silicone oil having an amino group and a silicone oil having a carboxyl group,
to an oil solution which may contain an oily substance to be gelatinized, followed by mixing; and
if necessary, further adding an oily substance to be gelatinized to the resulting mixture, followed by mixing.

13. A modified product of alkoxytitanium or alkoxyzirconium with a carboxylic acid and a hydroxy compound.

14. The modified product according to claim 13, wherein the hydroxy compound is at least one selected from an ether compound having a hydroxyl group, an ester compound having a hydroxyl group, an amino compound having a hydroxyl group, an amide compound having a hydroxyl group, and a polyhydric alcohol.
